# EUROPÄISCHE PATENTANMELDUNG

(11) **EP 1 238 968 A1**
(43) Veröffentlichungstag der Anmeldung: **11.09.2002**
(21) Anmeldenummer: 02003615.8
(22) Anmeldetag: 16.02.2002
(51) Int. Cl.: C07C 263/04, C07C 265/04, B01J 19/00

(54) **Verfahren zur Herstellung von organischen Polyisocyanaten**

(30) Priorität: 10.03.2001 DE 10111645
(71) Anmelder: BASF AKTIENGESELLSCHAFT, 67056 Ludwigshafen (DE)
(72) Erfinder: Klötzer, Matthias, Dr., 01945 Kroppen (DE); Krase, Volker, 01979 Lauchhammer (DE); Morgenschweis, Konrad, Dr., 01108 Dresden (DE); Pfab, Peter, Dr., 01987 Schwarzheide (DE); Schmidt, Andreas, Dr., 01987 Schwarzheide (DE); Starosta, Dieter, 01987 Schwarzheide (DE)
(74) Vertreter: Fitzner, Uwe, Dr.

(57) **Zusammenfassung**

Die vorliegende Erfindung betrifft ein Verfahren zur Herstellung von organischen Polyisocyanaten, bei welchem in einem Spaltreaktor Polyurethane thermisch gespalten, anschließend eine Destillation durchgeführt wird und die hierbei anfallende Sumpffraktion rückgeführt wird, wobei die rückgeführte Sumpffraktion wenigstens teilweise zunächst in einen Reaktionsbereich und anschließend in den Spaltreaktor geleitet wird.

## Beschreibung

Die vorliegende Erfindung betrifft ein Verfahren zur Herstellung von organischen Polyisocyanaten durch thermische Spaltung von Polyurethanen.

Die nach dem Stand der Technik bekannten phosgenfreien Herstellungsverfahren für organische Polyisocyanate basieren auf der Bildung von monomeren Polyurethanen aus Polyaminen und Kohlensäurederivaten und der anschließenden thermischen Spaltung der monomeren Polyurethane in Alkohole und Polyisocyanate.

Aus der EP 0 355 443 und 0 566 925 ist bekannt, daß die in der Urethanisierungsstufe gebildeten monomeren Polyurethane üblicherweise zunächst vom Alkohol und den niedrigsiedenden Nebenprodukten, wie Carbamaten und Carbonaten, destillativ befreit werden. Anschließend werden hochsiedende Komponenten entweder durch vollständige Destillation des gesamten Urethanstromes (EP 0 355 443) oder durch Destillation nur eines Teiles des Urethanstromes (EP 0 566 925) als verbleibende Sumpffraktion ausgeschleust.
Nach der EP 0 566 925 wird anschließend die Spaltung der monomeren Polyurethane bei 200 bis 300 °C und einem Druck von 1 bis 200 mbar unter Abdestillieren der flüchtigen Spaltprodukte kontinuierlich vorgenommen. Der ungespaltene Anteil der Reaktionsmischung sowie die gebildeten hochsiedenden Nebenprodukte bilden dabei eine Sumpffraktion, die in den Urethanisierungsprozeß zurückgeführt wird.

Die bei der anschließenden Reindestillation des Polyisocyanates anfallende Sumpffraktion enthält noch größere Mengen an nur teilweise gespaltenem, urethangruppenhaltigem Isocyanat und davon abgeleiteten Oligomeren. Solche Oligomere sind insbesondere Allophanate. Diese Sumpffraktion wird kontinuierlich ausgeschleust und in den Spaltreaktor zurückgeführt.

Diese Vorgehensweise hat den Nachteil, daß die teilweise gespaltenen, urethangruppenhaltigen Isocyanate unter den im Spaltreaktor herrschenden Bedingungen flüchtig sind und demgemäß unmittelbar verdampfen. Ein großer Teil des zurückgeführten urethangruppenhaltigen Isocyanates wird demgemäß nicht gespalten. Zusammen mit dem teilweise gespaltenen, urethangruppenhaltigen Isocyanat aus dem unteren Kolonnenrücklauf führt dies zu einem Ansteigen der stationären Menge an diesem Zwischenprodukt im Bereich unterhalb des Seitenabzuges in der Rektifikationskolonne. Dieses wiederum führt dazu, daß mehr teilweise gespaltenes, urethangruppenhaltiges Isocyanat am Seitenabzug entnommen und wiederum nach der anschließenden Isocyanatdestillation als Schwersieder in den Spaltreaktor zurückgeführt wird. Das führt letztlich nur zu einem erhöhten Energieaufwand im Spaltreaktor für diese zusätzliche Verdampferleistung, ohne eine effiziente Erhöhung der Polyisocyanat-Ausbeute erreichen zu können.

Aufgabe der vorliegenden Erfindung war es demgemäß, ein Verfahren zur Herstellung von organischen Polyisocyanaten, bei welchem in einem Spaltreaktor Polyurethane thermisch gespalten, anschließend eine Destillation durchgeführt wird und die hierbei anfallende Sumpffraktion rückgeführt wird, zur Verfügung zu stellen, das die beschriebenen Nachteile nicht aufweist. Mit diesem Verfahren soll insbesondere eine Methode zur Verfügung gestellt werden, mit der der Gehalt an teilweise gespaltenem, urethangruppenhaltigem Isocyanat am Seitenabzug der Rektifikationskolonne gesenkt und gleichzeitig die Ausbeute des Urethanspaltungsprozesses gesteigert wird.

Diese Aufgabe wird dadurch gelöst, daß die rückgeführte Sumpffraktion zunächst in einen Reaktionsbereich und anschließend in den Spaltreaktor geleitet wird.

In den Reaktionsbereich kann zusätzlich monomeres Polyurethan geführt werden. Erfindungsgemäß ist es möglich, entweder das gesamte monomere Polyurethan oder nur einen Teil des monomeren Polyurethans in den Reaktionsbereich zu führen, während der andere Teil direkt in den Spaltreaktor geleitet wird. Das Verhältnis von direkt in den Spaltreaktor geführtem Polyurethan zu in den Reaktionsbereich gegebenem Polyurethan richtet sich nach den jeweiligen Verfahrensbedingungen. Insbesondere hängt die Menge des zugegebenen Polyurethans von der Menge an rückgeführter Sumpffraktion ab. D.h., insbesondere beim Anfahren des Prozesses wird zunächst überwiegend Polyurethan direkt in den Spaltreaktor gegeben. Die Rückführung des teilweise gespaltenen, urethangruppenhaltigen Isocyanates kann vollständig oder auch nur teilweise vom Sumpf der Isocyanatreindestillation aus erfolgen. Zusätzlich oder alternativ kann auch eine Rückführung vollständig oder teilweise vom unteren Rücklauf der Rektifikationskolonne in den Spaltreaktor durchgeführt werden.

Sowohl das monomere Polyurethan als auch die Sumpffraktion können in einer bevorzugten Ausführungsform der Erfindung kontinuierlich in den Reaktionsbereich zurückgeführt werden.

Bei dem beschriebenen Einsatz des Reaktionsbereichs wird erfindungsgemäß somit erreicht, daß die zurückgeführte Sumpffraktion, welche nur teilweise gespaltenes, urethangruppenhaltiges Isocyanat enthält, vollständig oder teilweise mit dem zur Spaltung eingesetzten monomeren Polyurethanen vereint. Hierbei kommt es in dem Reaktionsbereich zu einer chemischen Umsetzung des zugeführten monomeren Polyurethans mit den aus der rückgeführten Sumpffraktion stammenden Isocyanaten. Im Ergebnis erfolgt teilweise oder vollständig eine Umsetzung zu Allophanaten.

Das in dem, der Spaltung vorgelagerten, Reaktionsbereich aus den monomeren Polyurethanen und den teilweise gespaltenen urethangruppenhaltigen Isocyanaten aus der rückgeführten Sumpffraktion gebildete, Allophanate enthaltende Gemisch wird dem Spaltreaktor zur Herstellung des Polyisocyanates zugeführt. Dieser Prozeß erfolgt vorzugsweise kontinuierlich.

Der Reaktionsbereich ist erfindungsgemäß vorzugsweise als beheizbarer Behälter ausgestaltet. Besonders bevorzugt ist die Ausgestaltung als separater Behälter. Dieser ist in einer bevorzugten Variante der Erfindung als Vorlagebehälter ausgestaltet, der im Zulauf zum Spaltreaktor angeordnet ist. Der beheizte Behälter wird zur besseren Durchmischung zweckmäßigerweise kontinuierlich gerührt und/oder durch eine Förderpumpe kontinuierlich umgewälzt. Der Reaktionsbereich kann jedoch auch in der Rückführleitung zur Rektifikationskolonne angeordnet sein. Daneben ist wenigstens eine gesonderte Zuführleitung für monomeres Polyurethan vorgesehen.

In einer weiteren Variante der Erfindung kann der Reaktionsbereich in die dem Spaltreaktor nachgeschalten Rektifizierkolonne integriert sein. Hierfür ist vorzugsweise ein geeigneter Einbau in der Rektifikationskolonne vorgesehen, in welchem die Bildung der Allophanate erfolgt. Dabei erfolgt die Allophanatbildung vorzugsweise auf einem Kolonnenboden mit definiertem Volumen und/oder in einem Rücklaufverteiler mit vergrößertem Volumen und/oder in einem separaten Verweilzeitreaktor, insbesondere einem Reaktor mit enger Verweilzeitverteilung. Die Allophanate entstehen dabei durch die Reaktion von urethangruppenhaltigen und isocyanatgruppenhaltigen Verbindungen aus dem Rücklauf der Rektifizierkolonne zur Abtrennung der Spaltprodukte unterhalb des Polyisocyanatseitenabzuges.

Eine weitere Alternative besteht darin, daß zusätzlich eine Rückführung der Sumpfströme aus der Polyisocyanatreindestillation in die Verweilzeitstrecken der dem Spaltreaktor nachgeschalteten Rektifizierkolonne erfolgt.

Für den Grad der Umsetzung entscheidend ist die Wahl der Reaktionsbedingungen. Sofern der Reaktionsbereich als dem Spaltreaktor vorgelagerter Behälter ausgestaltet wird, ist es erfindungsgemäß bevorzugt, in dem Reaktionsbereich Temperaturen zwischen 50 und 250 °C einzustellen. Besonders bevorzugt werden 60 bis 200 °C.

Weiterhin wird vorzugsweise eine Verweilzeit von 0,1-36h, bevorzugt von 1-12h eingestellt. Schließlich wird ein Druck von 0,1-10 bar, bevorzugt 1-5 bar eingehalten.

Sofern der Reaktionsbereich in die Rektifizierkolonne integriert wird, wird ein Temperaturbereich von 150-250 °C, insbesondere 180-240 °C bevorzugt. Der Druck liegt in diesem Fall vorzugsweise bei 1-200 mbar, insbesondere 10-100 mbar. Die Verweilzeit beträgt 0,1-20 min., vorzugsweise 0,5-5 min.

Die Allophanatbildung kann ggf. in Gegenwart eines oder mehrerer Katalysatoren erfolgen. Die zur Allophanatbildung verwendeten Katalysatoren entsprechen den im Verfahren zur Herstellung der entsprechenden monomeren Polyurethane und/oder deren thermischer Spaltung verwendeten Katalysatoren. Dabei handelt es sich vor allem um anorganische oder organische Metallverbindungen (Alkoholate, Acetylacetonate, Carboxylate, Halogenide und Pseudohalogenide) von Elementen
der Gruppen IIIa, IVa, Ib, IIb, IVb, VIb, VIIb, VIIIb (insbesondere Al-, Sn-, Cu-, Zn-, Ti-, Zr-, Mo-, Mn-, Fe-, Co-, Ni-Verbindungen).

Im folgenden wird die Erfindung anhand der Figuren näher beschrieben:
Figur 1 stellt eine Anlage nach dem Stand der Technik dar.
Figur 2 stellt die erfindungsgemäße Variante mit einem Vorlagebehälter dar.
Figur 3 stellt die erfindungsgemäße Variante mit speziellen Einbauten in der Rektifizierkolonne dar.

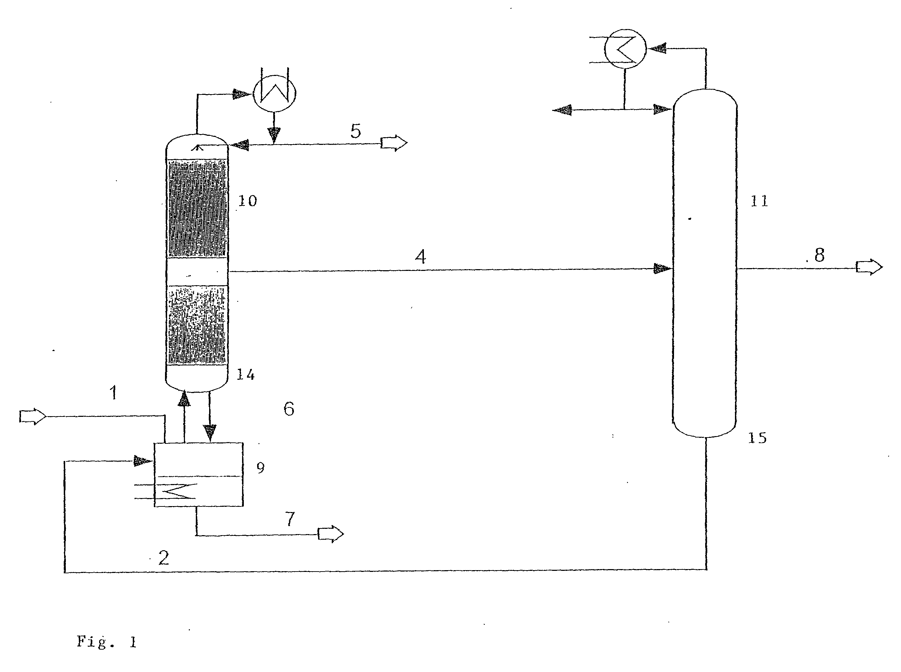

In Figur 1 ist eine Anlage nach dem Stand der Technik dargestellt. Hierbei wird über den Strom 1 monomeres Polyurethan in einen Spaltreaktor 9 geführt. In diesem Spaltreaktor 9 erfolgt die Spaltung der monomeren Polyurethane unter gleichzeitigem Abdestillieren der flüchtigen Spaltprodukte. Dem Spaltreaktor nachgeordnet ist eine Rektifizierkolonne 10. Über den Strom 4 wird das anfallende Polyisocyanat abgeführt. Der ungespaltene Anteil der Reaktionsmischung sowie die gebildeten hochsiedenden Nebenprodukte bilden die Sumpffraktion 15. Diese wird über den Strom 6 wieder dem Spaltreaktor zugeführt.
Das über den Strom 4 der Destillationskolonne 11 zugeführte Polyisocyanat wird einer Reindestillation unterworfen und über den Strom 8 abgeführt. Die anfallende Sumpffraktion enthält größere Mengen an teilweise gespaltenem, urethangruppenhaltigem Isocyanat und davon abgeleiteten Oligomeren. Diese Sumpffraktion 15 wird über den Strom 2 in den Spaltreaktor 9 zurückgeführt.

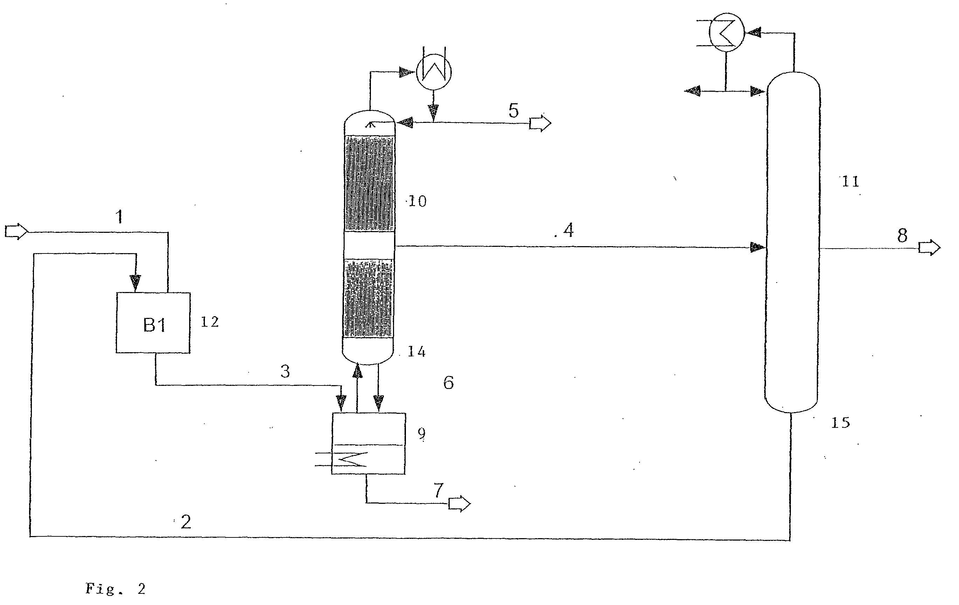

Die Anlage gemäß Figur 2 weist im wesentlichen denselben Aufbau wie Figur 1 auf. Der einzige Unterschied ist, daß die Monomeren über den Strom 1 nicht direkt in den Spaltreaktor 9 geführt werden. Vielmehr werden die Monomere zunächst in den Vorlagebehälter 12 geführt. Dieser von dem Spaltreaktor 9 getrennte Behälter ist beheizbar.

In den Vorlagebehälter wird ferner über den Strom 2 die Sumpffraktion 15 aus der Destillationskolonne 11 geführt. In dem Vorlagebehälter 12 werden somit die Sumpffraktion, welche nur teilweise gespalten ist, urethangruppenhaltiges Isocyanat enthält, vollständig oder wenigstens teilweise mit dem zur Spaltung eingesetzten monomeren Polyurethanen vereint. Hierbei kommt es in dem Vorlagebehälter 12 zu einer chemischen Umsetzung des mit dem Strom 1 zugeführten monomeren Polyurethans. Im Ergebnis erfolgt teilweise oder sogar vollständig eine Umsetzung zu Allophanaten. Wie bereits in dem allgemeinen Teil der Beschreibung angegeben wurde, werden in dem Vorlagebehälter 12 Temperaturen zwischen 12 und 250 °C und Drücke von 0,1 bis 10 bar eingestellt. Die Verweilzeit liegt regelmäßig zwischen 0,1 und 36 Std., bevor über den Strom 3 die Reaktionsprodukte in den Spaltreaktor 9 geführt werden.

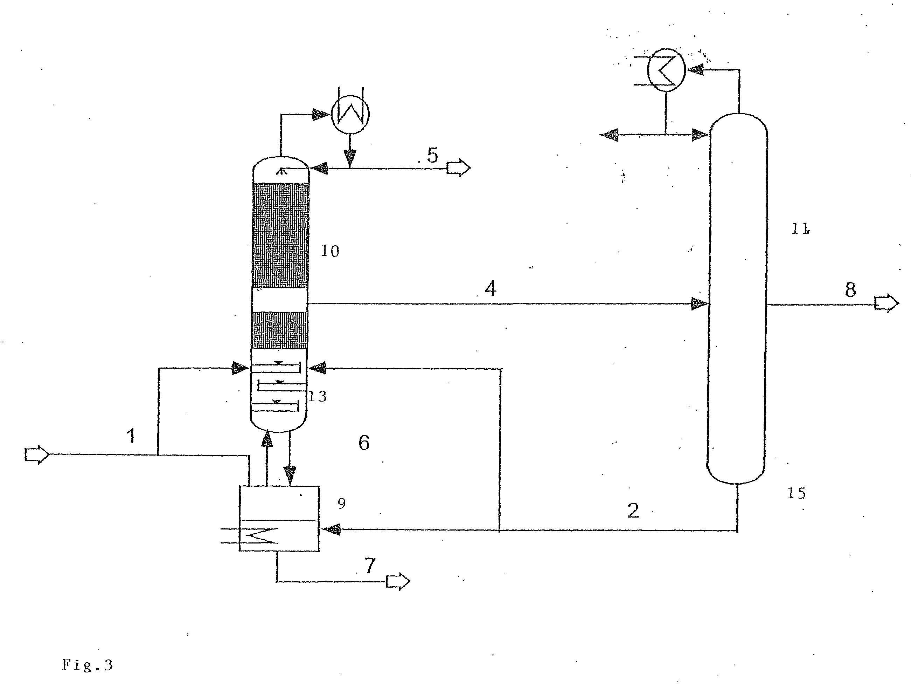

In Figur 3 ist schließlich als weitere Variante der Erfindung eine Anlage dargestellt, bei der Reaktionsbereich 13 in die dem Spaltreaktor 9 nachgeschaltete Rektifizierkolonne 10 integriert ist. Dementsprechend sind in dem Reaktionsbereich 13 Einbauten vorgesehen, in welchem die Bildung der Allophanate erfolgt. In der dargestellten Variante können die monomeren Polyurethane direkt in den Reaktionsbereich 13 geführt werden oder in den Spaltreaktor 9 gegeben werden. Ferner ist aus Figur 3 ersichtlich, daß die Sumpffraktion 15 über den Strom 2 entweder direkt in den Spaltreaktor gegeben wird oder in den Reaktionsbereich 13 geführt wird.

Im folgenden wird die Erfindung unter Bezugnahme auf die Beispiele näher beschrieben.

### Beispiel 1 (Vergleichsbeispiel)

Einem dampfbeheizten Verdampferreaktor mit 2,5 1 Reaktionsvolumen wurde über eine Dosiervorrichtung O-Butylcarbamat freies Hexamethylen-1,6-dibutylurethan, enthaltend 0,15 mol% Dibutylzinndilaurat, im schmelzflüssigem Zustand für die homogen katalysierte thermische Spaltung kontinuierlich zugeführt. Die Spaltung mit einem Umsatz von ca. 55 % bezüglich der eingesetzten 3,81 kg/h Hexamethylen-1,6-dibutylurethan erfolgte bei 30 mbar unter intensivem Sieden des Reaktionsgemisches. Die gasförmigen Brüden gelangten zur Auftrennung in eine Rektifizierkolonne, an deren Kopf 1,1 kg/h flüssiges Spaltbutanol abgenommen wurden. Im Seitenabzug fiel ein ca. 95 gew.%iges Rohdiisocyanat an. Ungespaltenes Diurethan und 6-Isocyanatohexyl-1-butylurethan wurden in den Verdampferreaktor zurückgeführt, aus dem kontinuierlich ein, hochsiedende Nebenprodukte enthaltender, Ablauf abgezogen wurde. Das so gewonnene Hexamethylen-1,6-diisocyanat wurde einer Reindestillation unterzogen, wobei im Seitenabzug einer bei 30 mbar betriebenen Kolonne 1,115 kg/h Hexamethylen-1,6-diisocyanat mit einer Reinheit von >99% anfiel. Der Sumpf der Reindestillation, der sich vorwiegend aus 6-Isocyanatohexyl-1-butylurethan und dessen höhermolekularen Oligomeren zusammensetzte, wurde direkt in den Verdampferreaktor bzw. in die anschließende Rektifizierkolonne zurückgeführt.

### Beispiel 2 (erfindungsgemäß)

Einem beheizten Verdampferreaktor mit 10 1 Reaktionsvolumen und einer Innentemperatur von ca. 130 °C wurden über eine Dosiervorrichtung O-Butylcarbamat freies Hexamethylen-1,6-dibutylurethan, enthaltend 0,15 mol% Dibutylzinndilaurat, im schmelzflüssigem Zustand sowie der bei der Reindestillation des Hexamythelen-1,6-Diisocyanat anfallende Sumpf im schmelzflüssigem Zustand kontinuierlich zugeführt. Das so erhaltene Gemisch wurde über eine Dosiereinrichtung kontinuierlich einem dampfbeheizten Verdampferreaktor mit 2,5 1 Reaktionsvolumen zur homogen katalysierten thermischen Spaltung zugeführt. Die Spaltung mit einem Umsatz von ca. 60 % bezüglich der eingesetzten 3,81 kg/h Hexamethylen-1,6-dibutylurethan erfolgte bei 30 mbar unter intensivem Sieden des Reaktionsgemisches. Die gasförmigen Brüden gelangten zur Auftrennung in eine Rektifizierkolonne, an deren Kopf 1,2 kg/h flüssiges Spaltbutanol abgenommen wurden. Im Seitenabzug fiel ein ca. 98 gew.%iges Rohdiisocyanat an. Ungespaltenes Diurethan und 6-Isocyanatohexyl-1-butylurethan wurden in den Verdampferreaktor zurückgeführt, aus dem kontinuierlich ein, hochsiedende Nebenprodukte enthaltender, Ablauf abgezogen wurde. Das so gewonnene Hexamethylen-1,6-diisocyanat wurde einer Reindestillation unterzogen, wobei im Seitenabzug einer bei 30 mbar betriebenen Kolonne 1,21 kg/h Hexamethylen-1,6-diisocyanat mit einer Reinheit von >99% anfiel. Der Sumpf der Reindestillation, der sich vorwiegend aus 6-Isocyanatohexyl-1-butylurethan und dessen höhermolekularen Oligomeren zusammensetzte, wurde direkt in den o.g. beheizten Reaktionsbereich zurückgeführt.

### Beispiel 3 (erfindungsgemäß)

Einem beheizten und kontinuierlich umgewälzten Vorlagebehälter mit 10 1 Reaktionsvolumen und einer Innentemperatur von 184 °C wurden über eine Dosiervorrichtung O-Butylcarbamat freies Hexamethylen-1,6-dibutylurethan, enthaltend 0,15 mol% Dibutylzinndilaurat, im schmelz-flüssigem Zustand, sowie der bei der Reindestillation des Hexamethylen-1,6-diisocyanat anfallende Sumpf im schmelzflüssigen Zustand kontinuierlich zugeführt. Das so erhaltene Gemisch wurde über eine Dosiereinrichtung kontinuierlich einem dampfbeheizten Verdampferreaktor mit 2,5 1 Reaktionsvolumen zur homogen katalysierten thermischen Spaltung zugeführt. Die Spaltung mit einem Umsatz von ca. 62 % bezüglich der eingesetzten 4,42 kg/h Hexamethylen-1,6-dibutylurethan erfolgte bei 30 mbar unter intensivem Sieden des Reaktionsgemisches. Die gasförmigen Brüden gelangten zur Auftrennung in eine Rektifizierkolonne, an deren Kopf 1,47 kg/h flüssiges Spaltbutanol abgenommen wurden. Im Seitenabzug fiel ein ca. 97 gewichtsprozentiges Rohdiisocyanat an. Ungespaltenes Diurethan und 6-Isocyanatohexyl-1-butylurethan wurden in den Verdampferreaktor zurückgeführt, aus dem kontinuierlich ein, hochsiedende Nebenprodukt enthaltender, Ablauf abgezogen wurde. Das so gewonnene Hexamethylen-1,6-diisocyanat wurde einer Reindestillation unterzogen, wobei im Seitenabzug einer bei 30 mbar betriebenen Kolonne 1,44 kg/h Hexamethylen-1,6-diisocyanat mit einer Reinheit von >99 % anfiel. Der Sumpf der Reindestillation, der sich vorwiegend aus 6-Isocyanatohexyl-1-butylurethan und dessen höhermolekularen Oligomeren zusammensetzte, wurde kontinuierlich direkt in den o.g. beheizten Vorlagebehälter zurückgeführt.

**Tabelle**

| | Beispiel 1 Vergleichsbeispiel | Beispiel 2 erfindungsgemäß | Beispiel 3 erfindungsgemäß |
|---|---|---|---|
| Zulauf (kg/h) Hexamethylen-1,6-dibutylurethan | 3,81 | 3,81 | 4,42 |
| Konzentration (Ma%) Hexamethylen-1,6-diisocyanat im Seitenabzug der Rektifikationskolonne | 95 | 98 | 97 |
| Sumpfrückführung (kg/h) Reindestillation | 0,10 | 0,10 | 0,12 |
| Temperatur Vorlagebehälter (°C) zur Allophanatbildung | Keine vorgeschaltete Allophanatbildung | 130 | 184 |
| Produktstrom (kg/h) Hexamethylen-1,6-diisocyanat | 1,115 | 1,21 | 1,44 |
| Umsatz bezüglich Hexamenthylen-1,6-dibutylurethan (Mol%) | 55 | 60 | 62 |

## Patentansprüche

1. Verfahren zur Herstellung von organischen Polyisocyanaten, bei welchem in einem Spaltreaktor Polyurethane thermisch gespalten, anschließend eine Destillation durchgeführt wird und die hierbei anfallende Sumpffraktion rückgeführt wird,
**dadurch gekennzeichnet, daß** die rückgeführte Sumpffraktion wenigstens teilweise zunächst in einen Reaktionsbereich und anschließend in den Spaltreaktor geleitet wird.

2. Verfahren nach Anspruch 1,
**dadurch gekennzeichnet, daß** zusätzlich monomeres Polyurethan in den Reaktionsbereich gegeben wird.

3. Verfahren nach einem der Ansprüche 1 oder 2,
**dadurch gekennzeichnet, daß** in den Reaktionsbereich Katalysatoren gegeben werden.

4. Verfahren nach Anspruch 3,
**dadurch gekennzeichnet, daß** die Katalysatoren anorganische oder organische Metallverbindungen von Elementen der Gruppen IIIa, IVa, Ib, IIb, IVb, VIb, VIIb, VIIIb sind.

5. Verfahren nach Anspruch 2,
**dadurch gekennzeichnet, daß** das monomere Polyurethan teilweise in den Spaltreaktor und teilweise in den Reaktionsbereich gegeben wird.

6. Verfahren nach einem der Ansprüche 1 bis 5,
**dadurch gekennzeichnet, daß** die rückgeführte Sumpffraktion und das monomere Polyurethan kontinuierlich in den Reaktionsbereich geleitet werden.

7. Verfahren nach einem der Ansprüche 1 bis 6,
**dadurch gekennzeichnet, daß** die Aufenthaltsdauer der Sumpffraktion und des monomeren Polyurethans und die Temperatur in dem Reaktionsbereich so eingestellt werden, daß wenigstens eine teilweise Umsetzung zu Allophanaten erfolgt.

8. Verfahren nach Anspruch 7,
**dadurch gekennzeichnet, daß** die Isocyanate aus der Sumpffraktion mit dem monomeren Polyurethan in dem Reaktionsbereich vollständig zu Allophanaten umgesetzt werden.

9. Verfahren nach einem der Ansprüche 1 bis 8,
**dadurch gekennzeichnet, daß** die Sumpffraktion über einen Reaktionsbereich geführt wird, der als beheizbarer, separater Behälter ausgestaltet ist.

10. Verfahren nach Anspruch 9,
**dadurch gekennzeichnet, daß** die Temperatur in dem Reaktionsbereich auf 50 bis 250 °C eingestellt wird.

11. Verfahren nach einem der Ansprüche 9 oder 10,
**dadurch gekennzeichnet, daß** die Temperatur in dem Reaktionsbereich auf 60 bis 200 °C eingestellt wird.

12. Verfahren nach einem der Ansprüche 9 bis 11,
**dadurch gekennzeichnet, daß** die Verweilzeit in dem Reaktionsbereich 0,1-36h beträgt.

13. Verfahren nach einem der Ansprüche 9 bis 12,
**dadurch gekennzeichnet, daß** die Verweilzeit in dem Reaktionsbereich 1-12h beträgt.

14. Verfahren nach einem der Ansprüche 9 bis 13,
**dadurch gekennzeichnet, daß** der Druck 0,1 bis 10 bar beträgt.

15. Verfahren nach einem der Ansprüche 9 bis 14,
**dadurch gekennzeichnet, daß** der Druck 1 bis 5 bar beträgt.

16. Verfahren nach einem der Ansprüche 1 bis 7,
**dadurch gekennzeichnet, daß** die Sumpffraktion über einen Reaktionsbereich geführt wird, der in die dem Spaltreaktor nachgeschaltete Reaktionskolonne integriert ist.

17. Verfahren nach Anspruch 16,
**dadurch gekennzeichnet, daß** die Temperatur 150 bis 250 °C beträgt.

18. Verfahren nach einem der Ansprüche 16 oder 17,
**dadurch gekennzeichnet, daß** der Druck 1 bis 200 mbar beträgt.

19. Verfahren nach einem der Ansprüche 16 bis 18,
**dadurch gekennzeichnet, daß** die Verweilzeit 0,1 bis 20 min. beträgt.

20. Vorrichtung mit wenigstens einem Spaltreaktor und wenigstens einer Rektifizierkolonne zur Herstellung von organischen Polyisocyanaten durch thermische Spaltung von Polyurethan und anschließende Destillation,
**dadurch gekennzeichnet, daß** in wenigstens einem Zulauf zu dem Spaltreaktor ein beheizbarer Reaktionsbereich angeordnet ist.

21. Vorrichtung nach Anspruch 20,
**dadurch gekennzeichnet, daß** der Reaktionsbereich in der Rückführleitung von der Rektifizierkolonne zum Spaltreaktor angeordnet ist.

22. Vorrichtung nach einem der Ansprüche 20,
**dadurch gekennzeichnet, daß** der Reaktionsbereich wenigstens eine Zuführleitung für monomeres Polyurethan enthält.

23. Vorrichtung nach einem der Ansprüche 20 bis 22,
**dadurch gekennzeichnet, daß** der Reaktionsbereich als beheizbarer, separater Behälter ausgestaltet ist.

24. Vorrichtung nach einem der Ansprüche 20 bis 23,
**dadurch gekennzeichnet, daß** der Reaktionsbereich in die dem Spaltreaktion nachgeschaltete Rektifikationskolonne integriert ist.

25. Verwendung der Vorrichtung nach einem der Ansprüche 20 bis 24 zur Herstellung von Polyisocyanaten.
